# EUROPEAN PATENT APPLICATION

(11) **EP 4 830 735 A2**
(43) Date of publication of application: **28.10.2026**
(21) Application number: 26190142.5
(22) Date of filing: 25.03.2020
(51) Int. Cl.: A61K 47/69

(54) **HUMAN PAPILLOMAVIRUS NANOPARTICLE FORMULATIONS**

(30) Priority: 26.03.2019 US 201962824227 P
(62) Document number(s) of the earlier application(s) in accordance with Art. 76 EPC: 24184083.4 / 4 420 731; 20718514.1 / 3 946 256
(71) Applicant: Aura Biosciences, Inc., Boston, MA 02135 (US)
(72) Inventor: MONKS, Stephen, A., Lexington, 02421 (US)
(74) Representative: Graham Watt & Co

(57) **Abstract**

The present disclosure provides, in some aspects, virus-like particle drug conjugate formulations and use of the conjugates for treating ocular tumors or lesions.

## Description

### RELATED APPLICATION

This application claims the benefit under 35 U.S.C. § 119(e) of U.S. provisional application number 62/824,227, filed March 26, 2019, which is incorporated by reference herein in its entirety.

### BACKGROUND

The basic concerns of biotherapeutic formulations, including ophthalmic formulations, are stability and structural integrity of the active molecule during transit and storage, following multiple freeze-thaw cycles, successful delivery of the drug to its site of action, and speed and cost-effectiveness of development and the final product. Numerous details come into play including analytical methods and testing protocols, containers and closures, delivery devices and dosage forms, excipients and stabilizers, and compatibility of ingredients.

### SUMMARY

The present disclosure provides, in some aspects, ophthalmic compositions comprising a virus-like particle (VLP) drug conjugate comprising photosensitive molecules conjugated to capsid proteins of a VLP and 2-(N-morpholino)ethanesulfonic acid (MES) or a pharmaceutically acceptable salt thereof. Surprisingly, the particular MES-based formulations provided herein eliminate visible aggregation and precipitation of the VLP drug conjugate, relative to other formulations tested, in some embodiments, without at a low salt concentration and/or with a pH that is suitable for ophthalmic administration (e.g., less than 7, such 6.5).

Thus, provided herein, in some aspects, are ophthalmic compositions comprising a near-isotonic solution (255-345 mOsm/L) of a VLP drug conjugates comprising photosensitive molecules conjugated to capsid proteins of a VLP, wherein the VLP drug conjugates are in suspension. As used herein, an isotonic solution has an isotonicity of approximately 290 mOsm/L, and a near-isotonic solution has an isotonicity of 255-345 mOsm/L.

In some aspects, the ophthalmic compositions comprise VLP drug conjugates comprising photosensitive molecules conjugated to capsid proteins of a VLP, wherein the VLP drug conjugates do not aggregate to form visible particulate.

In some embodiments, the ophthalmic compositions further comprise at least one protective reagent and at least one surfactant. In some embodiments, the ophthalmic compositions further comprise at least one, at least two, or at least three reagent(s) selected from trehalose dihydrate, magnesium chloride (MgCl₂), sodium chloride (NaCl), and polysorbate 80 (PS80). In some embodiments, the ophthalmic compositions further comprise trehalose dihydrate, MgCl₂, NaCl, and PS80.

In some embodiments, the compositions comprise 0.01% to 0.1% (w/v) VLP drug conjugate. For example, the compositions may comprise 0.04% (w/v) VLP drug conjugate.

In some embodiments, the compositions comprise 0.1% to 1.0% (w/v) MES or a pharmaceutically acceptable salt thereof (e.g., MES hemisodium salt). For example, the compositions may comprise 0.4% (w/v) MES or a pharmaceutically acceptable salt thereof. In some embodiments, the compositions comprise 5 mM to 50 mM MES or a pharmaceutically acceptable salt thereof. For example, the compositions may comprise 20 mM MES or a pharmaceutically acceptable salt thereof.

In some embodiments, the compositions further comprise 1 % to 10% (w/v) trehalose dihydrate. For example, the compositions may further comprise 5% (w/v) trehalose dihydrate.

In some embodiments, the compositions further comprise 0.1% to 1.0% (w/v) NaCl. For example, the compositions may further comprise 0.37% (w/v) NaCl. In some embodiments, the compositions further comprise 20 mM to 100 mM NaCl. For example, the compositions may further comprise 63 mM NaCl.

In some embodiments, the compositions further comprise 0.1% to 1.0% (w/v) MgCl₂. For example, the compositions may further comprise 0.2% (w/v) MgCl₂. In some embodiments, the compositions further comprise 5 mM to 25 mM MgCl₂. For example, the compositions may further comprise 10 mM MgCl₂.

In some embodiments, the compositions further comprise 0.01% to 0.1% (w/v) polysorbate 80. For example, the compositions may further comprise 0.05% (w/v) polysorbate 80.

In some embodiments, the compositions have a pH value of 5 to 8. For example, the compositions may have a pH value of 6.5.

In some aspects, the ophthalmic compositions comprise 0.43% (w/v) MES, 5% (w/v) trehalose dihydrate, 0.37% (w/v) NaCl, 0.2% MgCl₂, 0.05% (w/v) polysorbate 80, and 0.04% (w/v) virus-like particle (VLP) drug conjugate, wherein the VLP drug conjugate comprises photosensitive molecules conjugated to capsid proteins of a VLP. In some aspects the ophthalmic compositions comprise 20 mM MES, 5% (w/v) trehalose dihydrate, 63 mM NaCl, 10 mM MgCl₂, 0.05% (w/v) polysorbate 80, and 0.04% (w/v) VLP drug conjugate, wherein the VLP drug conjugate comprises photosensitive molecules conjugated to capsid proteins of a VLP.

In some embodiments, the photosensitive molecules comprise infrared or near-infrared (e.g., phthalocyanine) dye molecules. For example, the photosensitive dye molecules may comprise IRDye^{®} 700DX molecules, IRDye^{®} 800CW molecules, or a mixture of IRDye^{®} 700DX and IRDye^{®} 800CW molecules. Other therapeutic and diagnostic photosensitive molecules are contemplated herein.

In some embodiments, the VLPs comprise 10-1000 photosensitive molecules, 10-500 photosensitive molecules, 50-1000 photosensitive molecules, 50-500 photosensitive molecules, 100-1000 photosensitive molecules, or 100-500 photosensitive molecules. In some embodiments, the VLPs comprise 300 photosensitive molecules.

In some embodiments, the VLP comprises papillomavirus capsid proteins (e.g., human papillomavirus capsid proteins). For example, the papillomavirus capsid proteins may comprise L1 capsid proteins, L2 capsid proteins, or a combination of L1 and L2 capsid proteins. In some embodiments, the L1 capsid proteins are modified to reduce immunogenicity of the VLP.

Also provided herein, in some aspects, are methods that comprise administering to an eye of a subject an ophthalmic composition of the present disclosure, wherein the subject has ocular cancer (e.g., ocular melanoma), and wherein the ophthalmic solution is administered in an amount effective to treat the ocular melanoma. In some embodiments, the ophthalmic composition is administered by intravitreal injection. In some embodiments, the subject has an uveal melanoma. In some embodiments, the subject has a choroidal melanoma.

Further provided herein, in some aspects, are methods that comprise administering to an eye of a subject an ophthalmic composition of the present disclosure, wherein the subject has an indeterminate lesion, and wherein the ophthalmic solution is administered in an amount effective to treat the indeterminate lesion.

### BRIEF DESCRIPTION OF DRAWINGS

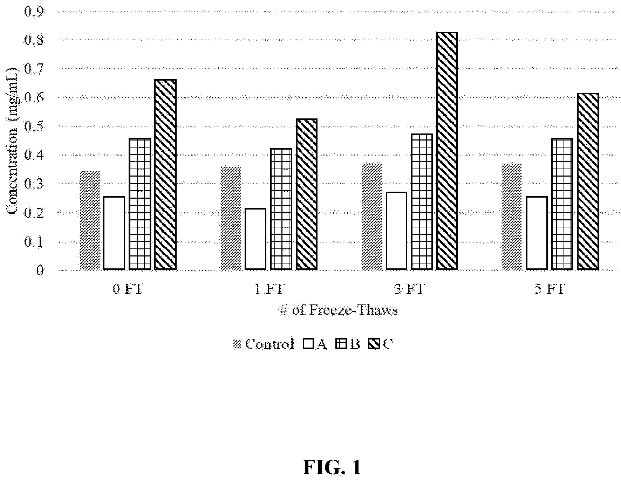

**FIG. 1** illustrates the recovery of protein per formulation as measured by a Bradford total protein assay. The control formulation contains 20 mM Potassium Phosphate, 500 mM NaCl, pH 7.0. Formulation A contains 20mM Potassium Phosphate, 5% (w/v) trehalose dihydrate, 10 mM MgCl₂, 63 mM NaCl, 0.05% (w/v) PS80, pH 7.0. Formulation B contains 50 mM HEPES, 5% (w/v) trehalose dihydrate, 10 mM MgCl₂, 47 mM NaCl, 0.05% (w/v) PS80, pH 7.5. Formulation C contains 20 mM MES, 5%(w/v) trehalose dihydrate, 10 mM MgCl₂, 63 mM NaCl, 0.05% (w/v) PS80, pH 6.5. The number of freeze-thaws (FT) is the number of times the sample was frozen and thawed.
**FIG. 2** illustrates the recovery of protein per formulation as measured by UV-Vis. The formulations are as in FIG. 1. The UV-Vis measures absorbance at 280 nm. The number of freeze-thaws (FT) is the number of times the sample was frozen and thawed.
**FIG. 3** shows a representative SDS-PAGE gel of protein formulations after 5 freeze-thaw cycles. The formulations are as in FIG. 1. The band at ~55 kDa is the L1 protein in the virus-like particles (VLPs).
**FIG. 4** shows representative transmission electron micrographs (TEM) images of virus-like particle drug conjugate (VLP drug conjugate). The formulations are as in FIG. 1.
**FIG. 5** illustrates the particle size distribution of VLP drug conjugate as measured by transmission electron microscopy (TEM). The formulations are as in FIG. 1. The number of freeze-thaws (FT) is the number of times the sample was frozen and thawed.
**FIG. 6** illustrates the average particle size of VLP drug conjugate as measured by dynamic light scattering (DLS). The formulations are as in FIG. 1. The average particle size is measured by the diameter. The number of freeze-thaws (FT) is the number of times the sample was frozen and thawed. N is the mean diameter by number, V is the mean diameter by volume, and I is the mean diameter by intensity.
**FIGS. 7A-7D** illustrate *in vitro* EC₅₀ killing curves. The formulations are as in **FIG. 1****.** **FIG.** 7A shows the percent (%) dead cells using VLP drug conjugate that had not been frozen (0 FT). **FIG. 7B** shows the % dead cells using VLP drug conjugate that had been frozen 1 time (1 FT). **FIG. 7C** shows the % dead cells using VLP drug conjugate that had been frozen 3 times. **FIG. 7D** shows the % dead cells using VLP drug conjugate that had been frozen 5 times.
**FIG. 8** illustrates a conjugation-formulation work-flow diagram. The formulations are as in **FIG. 1****.**

### DETAILED DESCRIPTION

The present disclosure provides, in some aspects, ophthalmic compositions comprising a virus-like particle (VLP) drug conjugate comprising photosensitive molecules conjugated to capsid proteins of a VLP and 2-(N-morpholino)ethanesulfonic acid (MES). The chemical structure of MES contains a morpholine ring, has a molecular weight of 195.2, and the chemical formula is C₆H₁₃NO₄S. In some embodiments, the compositions comprise 0.1% to 1.0% (w/v) MES or a pharmaceutically acceptable salt thereof (e.g., MES hemisodium salt). For example, the compositions may comprise 0.1% to 0.9%, 0.1% to 0.8%, 0.1% to 0.7%, 0.1% to 0.6%, 0.1% to 0.5%, 0.1% to 4%, 0.2% to 1.0%, 0.2% to 0.9%, 0.2% to 0.8%, 0.2% to 0.7%, 0.2% to 0.6%, 0.2% to 0.5%, 0.2% to 0.4%, 0.3% to 1.0%, 0.3% to 0.9%, 0.3% to 0.8%, 0.3% to 0.7%, 0.3% to 0.6%, 0.3% to 0.5%, 0.3% to 0.4%, 0.4% to 1.0%, 0.4% to 0.9%, 0.4% to 0.8%, 0.4% to 0.7%, 0.4% to 0.6%, or 0.4% to 0.5% (w/v) MES. In some embodiments, the compositions may comprise 0.1%, 0.2%, 0.3%, 0.4%, 0.5%, 0.6%, 0.7%, 0.8%, 0.9%, or 1% (w/v) MES. In some embodiments, the compositions may comprise 0.41%, 0.42%, 0.43%. 0.44%, or 0.45% (w/v) MES. In some embodiments, the compositions may comprise 0.43% (w/v) MES. In some embodiments, the compositions comprise 5 mM to 50 mM MES. For example, the compositions may comprise 5 mM to 45 mM, 5 mM to 40 mM, 5 mM to 35 mM, 5 mM to 30 mM, 5 mM to 25 mM, 5 mM to 20 mM, 10 mM to 50 mM, 10 mM to 45 mM, 10 mM to 40 mM, 10 mM to 35 mM, 10 mM to 30 mM, 10 mM to 25 mM, 10 mM to 20 mM, 20 mM to 50 mM, 20 mM to 45 mM, 20 mM to 40 mM, 20 mM to 35 mM, 20 mM to 30 mM, or 20 mM to 25 mM MES. In some embodiments, the compositions comprise 5 mM, 10 mM, 15 mM, 20 mM, 25 mM, 30 mM, 35 mM, 40 mM, 45 mM, or 50 mM MES. In some embodiments, the compositions comprise 15 mM, 16 mM, 17 mM, 18 mM, 19 mM, 20 mM, 21 mM, 22 mM, 23 mM, 24 mM, or 25 mM MES. In some embodiments, the compositions comprise 20 mM MES.

In some embodiments, the compositions comprise 0.01% to 0.1% (w/v) VLP drug conjugate. For example, the compositions may comprise 0.01% to 0.09%, 0.01% to 0.08%, 0.01% to 0.07%, 0.01% to 0.06%, 0.01% to 0.05%, 0.01% to 0.04%, 0.02% to 0.1%, 0.02% to 0.09%, 0.02% to 0.08%, 0.02% to 0.07%, 0.02% to 0.06%, 0.02% to 0.05%, 0.02% to 0.04%, 0.03% to 0.1%, 0.03% to 0.09%, 0.03% to 0.08%, 0.03% to 0.07%, 0.03% to 0.06%, 0.03% to 0.05%, 0.03% to 0.04%, 0.04% to 0.1%, 0.04% to 0.09%, 0.04% to 0.08%, 0.04% to 0.07%, 0.04% to 0.06%, or 0.04% to 0.05% (w/v) VLP drug conjugate. In some embodiments, the compositions may comprise 0.01%, 0.02%, 0.03%, 0.04%, 0.05%, 0.06%, 0.07%, 0.08%, 0.09%, or 0.1% (w/v) VLP drug conjugate. In some embodiments, the compositions may comprise 0.03%, 0.035%, 0.04%, 0.045%, or 0.05% (w/v) VLP drug conjugate. In some embodiments, the compositions may comprise 0.04% (w/v) VLP drug conjugate.

In some embodiments, the compositions comprise 0.01% to 0.2% (w/v) VLP drug conjugate. In some embodiments, the compositions comprise 0.01% to 0.3% (w/v) VLP drug conjugate. In some embodiments, the compositions comprise 0.01% to 0.4% (w/v) VLP drug conjugate. In some embodiments, the compositions comprise 0.01% to 0.5% (w/v) VLP drug conjugate. In some embodiments, the compositions comprise 0.1%, 0.2%, 0.3%, 0.4%, or 0.5% (w/v) VLP drug conjugate.

In some embodiments, the ophthalmic compositions further comprise at least one, at least two, or at least three reagent(s) selected from trehalose dihydrate, magnesium chloride (MgCl₂), sodium chloride (NaCl), and polysorbate 80 (PS80). In some embodiments, the ophthalmic compositions further comprise trehalose dihydrate and MgCl₂. In some embodiments, the ophthalmic compositions further comprise trehalose dehydrate and NaCl. In some embodiments, the ophthalmic compositions further comprise trehalose dehydrate and PS80. In some embodiments, the ophthalmic compositions further comprise MgCl₂ and NaCl. In some embodiments, the ophthalmic compositions further comprise MgCl₂ and PS80. In some embodiments, the ophthalmic compositions further comprise NaCl and PS80. In some embodiments, the ophthalmic compositions further comprise trehalose dihydrate, MgCl₂ and NaCl. In some embodiments, the ophthalmic compositions further comprise trehalose dihydrate, MgCl₂ and PS80. In some embodiments, the ophthalmic compositions further comprise trehalose dihydrate, NaCl, and PS80. In some embodiments, the ophthalmic compositions further comprise MgCl₂, NaCl, and PS80. In some embodiments, the ophthalmic compositions further comprise trehalose dihydrate, MgCl₂, NaCl, and PS80.

Trehalose is a disaccharide formed by a 1,1-glycosidic bond between two α-glucose units. Trehalose forms a rhomboid crystal as a dihydrate, and anhydrous forms of trehalose readily regain moisture to form the dihydrate. As shown herein, this excipient has a significant positive effect on VLP drug conjugate recovery and stability in MES buffer containing NaCl and MgCl₂. In some embodiments, the compositions further comprise 1 % to 10% (w/v) trehalose dihydrate. For example, the compositions may comprise 1% to 9%, 1% to 8%, 1% to 7%, 1% to 6%, 1% to 5%, 2% to 10%, 2% to 9%, 2% to 8%, 2% to 7%, 2% to 6%, 2% to 5%, 3% to 10%, 3% to 9%, 3% to 8%, 3% to 7%, 3% to 6%, 3% to 5%, 3% to 4%, 4% to 10%, 4% to 9%, 4% to 8%, 4% to 7%, 4% to 6%, or 4% to 5%, 5% to 10%, 5% to 9%, 5% to 8%, 5% to 7%, or 5% to 6% (w/v) trehalose dihydrate. In some embodiments, the compositions further comprise 1%, 2%, 3%, 4%, 5%, 6%, 7%, 8%, 9%, or 10% trehalose (w/v) dihydrate. In some embodiments, the compositions further comprise 4%, 4.5%, 5%, 5.5%, or 6% trehalose dihydrate. In some embodiments, the compositions further comprise 5% trehalose dihydrate.

In some embodiments, the compositions further comprise a salt, such as sodium chloride (NaCl), for isotonicity. In some embodiments, the compositions further comprise 0.1% to 1.0% NaCl. For example, the compositions may comprise 0.1% to 0.9%, 0.1% to 0.8%, 0.1% to 0.7%, 0.1% to 0.6%, 0.1% to 0.5%, 0.1% to 4%, 0.2% to 1.0%, 0.2% to 0.9%, 0.2% to 0.8%, 0.2% to 0.7%, 0.2% to 0.6%, 0.2% to 0.5%, 0.2% to 0.4%, 0.3% to 1.0%, 0.3% to 0.9%, 0.3% to 0.8%, 0.3% to 0.7%, 0.3% to 0.6%, 0.3% to 0.5%, 0.3% to 0.4%, 0.4% to 1.0%, 0.4% to 0.9%, 0.4% to 0.8%, 0.4% to 0.7%, 0.4% to 0.6%, or 0.4% to 0.5% (w/v) NaCl. In some embodiments, the compositions may comprise 0.1%, 0.2%, 0.3%, 0.4%, 0.5%, 0.6%, 0.7%, 0.8%, 0.9%, or 1% (w/v) NaCl. In some embodiments, the compositions further comprise 0.35%, 0.35%, 0.37%, 0.38%, or 0.39% (w/v) NaCl. In some embodiments, the compositions further comprise 0.37% (w/v) NaCl. In some embodiments, the compositions further comprise 20 mM to 100 mM NaCl. For example, the compositions may further comprise 20 mM to 90 mM, 20 mM to 80 mM, 20 mM to 70 mM, 20 mM to 60 mM, 20 mM to 50 mM, 30 mM to 100 mM, 30 mM to 90 mM, 30 mM to 80 mM, 30 mM to 70 mM, 30 mM to 60 mM, 30 mM to 50 mM, 40 mM to 100 mM, 40 mM to 90 mM, 40 mM to 80 mM, 40 mM to 70 mM, 40 mM to 60 mM, 40 mM to 50 mM, 50 mM to 100 mM, 50 mM to 90 mM, 50 mM to 80 mM, 50 mM to 70 mM, 50 mM to 60 mM, or 60 mM to 70 mM NaCl. In some embodiments, the compositions further comprise 60 mM, 61 mM, 62 mM, 63 mM, 64 mM, 65 mM, or 66 mM NaCl. In some embodiments, the compositions further comprise 63 mM NaCl.

In some embodiments, the compositions further comprise 0.1% to 1.0% (w/v) magnesium chloride (MgCl₂). For example, the compositions may comprise 0.1% to 0.9%, 0.1% to 0.8%, 0.1% to 0.7%, 0.1% to 0.6%, 0.1% to 0.5%, 0.1% to 4%, 0.2% to 1.0%, 0.2% to 0.9%, 0.2% to 0.8%, 0.2% to 0.7%, 0.2% to 0.6%, 0.2% to 0.5%, 0.2% to 0.4%, 0.3% to 1.0%, 0.3% to 0.9%, 0.3% to 0.8%, 0.3% to 0.7%, 0.3% to 0.6%, 0.3% to 0.5%, 0.3% to 0.4%, 0.4% to 1.0%, 0.4% to 0.9%, 0.4% to 0.8%, 0.4% to 0.7%, 0.4% to 0.6%, or 0.4% to 0.5% (w/v) MgCl₂. In some embodiments, the compositions may comprise 0.1%, 0.2%, 0.3%, 0.4%, 0.5%, 0.6%, 0.7%, 0.8%, 0.9%, or 1% (w/v) MgCl₂. In some embodiments, the compositions further comprise 0.1%, 0.15%, 0.2%, 0.25%, or 3% (w/v) MgCl₂. In some embodiments, the compositions further comprise 0.2% (w/v) MgCl₂. In some embodiments, the compositions further comprise 5 mM to 25 mM MgCl₂. For example, the compositions may comprise 5 mM to 20 mM, 5 mM to 15 mM, 5 mM to 10 mM, 10 mM to 25 mM, 10 mM to 20 mM, or 10 mM to 15 mM MgCl₂. In some embodiments, the compositions further comprise 5 mM, 10 mM, 15 mM, 20 mM, or 25 mM MgCl₂. In some embodiments, the compositions further comprise 8 mM, 9 mM, 10 mM, 11 mM, or 12 mM MgCl₂. In some embodiments, the compositions further comprise 10 mM MgCl₂.

In some embodiments, the compositions further comprise a surfactant, for example, to increase protein recovery and stability. In some embodiments, the compositions further comprise 0.01% to 0.1% polysorbate 80. For example, the compositions may further comprise 0.05% polysorbate 80. For example, the compositions may comprise 0.01% to 0.09%, 0.01% to 0.08%, 0.01% to 0.07%, 0.01% to 0.06%, 0.01% to 0.05%, 0.01% to 0.04%, 0.02% to 0.1%, 0.02% to 0.09%, 0.02% to 0.08%, 0.02% to 0.07%, 0.02% to 0.06%, 0.02% to 0.05%, 0.02% to 0.04%, 0.03% to 0.1%, 0.03% to 0.09%, 0.03% to 0.08%, 0.03% to 0.07%, 0.03% to 0.06%, 0.03% to 0.05%, 0.03% to 0.04%, 0.04% to 0.1%, 0.04% to 0.09%, 0.04% to 0.08%, 0.04% to 0.07%, 0.04% to 0.06%, or 0.04% to 0.05% (w/v) polysorbate 80. In some embodiments, the compositions may comprise 0.01%, 0.02%, 0.03%, 0.04%, 0.05%, 0.06%, 0.07%, 0.08%, 0.09%, or 0.1% (w/v) polysorbate 80. In some embodiments, the compositions may 0.04%, 0.045%, 0.05%, 0.055%, or 0.06% (w/v) polysorbate 80. In some embodiments, the compositions may comprise 0.05% (w/v) polysorbate 80.

In some embodiments, the compositions have a pH value of 5 to 8. For example, the compositions may have a pH value of 5 to 7 or 6 to 8. In some embodiments, the compositions have a pH value of 6.0, 6.1, 6.2, 6.3, 6.4, 6.5, 6.6., 6.7, 6.8, 6.9, or 7.0. In some embodiments, the compositions have a pH value of 6.5.

In some aspects, the ophthalmic compositions comprise 0.43% (w/v) MES, 5% (w/v) trehalose dihydrate, 0.37% (w/v) NaCl, 0.2% (w/v) MgCl₂, 0.05% (w/v) polysorbate 80, and 0.04% (w/v) virus-like particle (VLP) drug conjugate, wherein the VLP drug conjugate comprises photosensitive molecules conjugated to capsid proteins of a VLP. In some aspects the ophthalmic compositions comprise 20 mM MES, 5% (w/v) trehalose dihydrate, 63 mM NaCl, 10 mM MgCl₂, 0.05% (w/v) polysorbate 80, and 0.04% (w/v) VLP drug conjugate, wherein the VLP drug conjugate comprises photosensitive molecules conjugated to capsid proteins of a VLP.

In some embodiments, the formulation is visibly free from particulates. Visibly free from particulates means that a normal person (e.g., not having an uncorrected vision deficiency, not using a means to magnify the sample) does not perceive the presence of particulates (e.g., clumps, aggregates) in the formulation.

The compositions provided herein, in some embodiments, include at least one pharmaceutically-acceptable excipient *(e.g.,* carrier, buffer, and/or salt, *etc.).* A molecule or other substance/agent is considered "pharmaceutically acceptable" if it is approved or approvable by a regulatory agency of the Federal government or a state government or listed in the U.S. Pharmacopeia or other generally recognized pharmacopeia for use in animals, including humans. An excipient may be any inert (inactive), non-toxic agent, administered in combination with an agent provided herein. Non-limiting examples of excipients include buffers (e.g., sterile saline), salts, carriers, preservatives, fillers, surfactants, and coloring agents.

The term "pharmaceutically acceptable salt" refers to those salts which are, within the scope of sound medical judgment, suitable for use in contact with the tissues of humans and lower animals without undue toxicity, irritation, allergic response, and the like, and are commensurate with a reasonable benefit/risk ratio. Pharmaceutically acceptable salts are well known in the art. For example, Berge et al. describe pharmaceutically acceptable salts in detail in J. Pharmaceutical Sciences, 1977, 66, 1-19, incorporated herein by reference. Pharmaceutically acceptable salts of the compounds of the present disclosure include those derived from suitable inorganic and organic acids and bases. Examples of pharmaceutically acceptable, nontoxic acid addition salts are salts of an amino group formed with inorganic acids, such as hydrochloric acid, hydrobromic acid, phosphoric acid, sulfuric acid, and perchloric acid or with organic acids, such as acetic acid, oxalic acid, maleic acid, tartaric acid, citric acid, succinic acid, or malonic acid or by using other methods known in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium, and N+(C1-4 alkyl)4- salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

The ophthalmic composition, in some embodiments, is formulated as a solution. The solution may be packaged in a vial or a pre-filled syringe, for example. Other packaging and delivery forms are contemplated herein.

### Virus-like Particle Conjugates

### Virus-like Particles (VLPs)

In some embodiments, a VLP is a papillomavirus VLP. The VLP may be a human papillomavirus VLP (e.g., derived from a virus that can infect human), while in other embodiments, the VLP is a non-human papillomavirus VLP. Examples of non-human VLPs include those derived from, without limitation, bovine papillomaviruses, murine papillomaviruses, cotton-rabbit papillomaviruses and macaque or rhesus papillomaviruses. In some embodiments, the VLPs are bovine papillomavirus VLPs (e.g., assembled from BPV L1 capsid proteins or a combination of BPV L1 and BPV L2 capsid proteins).

A capsid protein is a protein monomer, several of which form a capsomer oligomer. A capsomer is the basic oligomeric structural unit of a viral capsid, which is an outer covering of protein that protects the genetic material of a virus such as, for example, human papillomavirus (HPV). The capsid proteins of the present disclosure include papillomavirus L1 major capsid proteins and papillomavirus L2 minor capsid proteins. In some embodiments, the VLPs of the present disclosure contain only L1 capsid proteins, while in other embodiments, the VLPs contain a mixture (or combination) of L1 and L2 capsid proteins. In some embodiments, a VLP comprises human papillomavirus capsid proteins. In some embodiments, a VLP comprises non-human papillomavirus capsid proteins.

In some embodiments, the percentage of L1 capsid proteins in a VLP is greater than the percentage of L2 capsid proteins in the VLP. For example, in some embodiments, the percentage of L1 capsid proteins in a VLP is 80% to 100% (of the total number of capsid proteins in the virus-like particle). In some embodiments, the percentage of L1 capsid proteins in a VLP is 80%, 85%, 90%, 91%, 92%, 93%, 94%, 95%, 96%, 97%, 98%, 99% or 100%. In some embodiments, the percentage of L2 capsid proteins in a VLP is 1% to 25% (of the total number of capsid proteins in the VLP). For example, some embodiments, the percentage of L2 capsid proteins in a VLP is 1%, 2%, 3%, 4%, 5%, 6%, 7%, 8%, 9%, 10%, 11%, 12%, 13%, 14%, 15%, 16%, 17%, 18%, 19% or 20%.

In some embodiment, a VLP contains 12 to 72 L2 proteins. In some embodiment, a VLP contains 360 L1 proteins and 12 to 72 L2 proteins. In some embodiments, capsid proteins assemble into VLPs having a diameter of 20 to 60 nm. For example, capsid proteins may assemble into VLPs having a diameter of 20, 25, 30, 35, 40, 45, 50, 55 or 60 nm.

VLPs in accordance with the present disclosure may have a modified immunogenicity and/or antigenicity with respect to the wild type papillomavirus VLPs. The VLPs may, for example, be assembled from capsomers having a variant capsid protein with modified immunogenicity and/or antigenicity. A variant capsid protein with "modified immunogenicity and/or antigenicity" is one that is modified naturally or synthetically (e.g., mutated, substituted, deleted, pegylated or inserted) at an amino acid to reduce or prevent recognition of the capsid protein by pre-existing (e.g., endogenous) viral serotype-specific antibodies. A variant capsid protein may be a human papillomavirus (HPV) L1 variant, a non-human papillomavirus L1 variant, or a papillomavirus L1 variant based on a combination of amino acids from different HPV serotypes. For example, an L1 variant with modified immunogenicity and/or antigenicity may be a recombinant protein based on HPV serotype 16 and HPV serotype 31 (referred to herein as a "variant HPV16/31 L1 protein"), which is described in International Pub. Nos. WO 2010/120266, WO 2013/119877, and WO 2015/042325, the entirety of each of which is incorporated by reference herein.

### Photosensitive Molecules

In accordance with various aspects of the present disclosure, photosensitive molecules may be conjugated to capsid proteins *(e.g.,* L1 and/or L2 capsid proteins) of the VLPs. In some embodiments, the photosensitive molecules are covalently conjugated to capsid proteins of the VLPs. In some embodiments, the photosensitive molecules are covalently conjugated to lysine residues of capsid proteins of the VLPs. VLPs that are conjugated to photosensitive molecules may be referred to herein as "VLP drug conjugates." In some embodiments, the photosensitive molecules comprise an NHS (N-Hydroxysuccinimide) ester group that reacts with an amine group of the capsid protein *(e.g.,* amine group of lysine or other amino acid) to form a covalent amide bond.

The ratio of photosensitive molecule (PM) to VLP may vary. In some embodiments the ratio of VLP:PM is about 1:10 to about 1:1000, about 1:10 to about 1:500, about 1:50 to about 1:500, or about 1:50 to about 1:1000. That it, in some embodiments, a VLP may comprise about 10 to about 1000 photosensitive molecules (e.g., of the same type or a mixture of different types). In some embodiments, the ratio of VLP:PM is 1:10, 1:15, 1:20, 1:25, 1:50, 1:75, 1:100, 1:150, 1:200, 1:250, 1:300, 1:350, 1:400, 1:450, 1:500, 1:550, 1:600, 1:650, 1:700, 1:750, 1:800. 1:850, 1:900, 1:950 or 1:1000. In some embodiments, the VLP may comprise 10-1000, 10-900, 10-800, 10-700, 10-600, 10-500, 10-400, 10-300, 10-200, 10-100, 20-1000, 20-900, 20-800, 20-700, 20-600, 20-500, 20-400, 20-300, 20-200, 20-100, 30-1000, 30-900, 30-800, 30-700, 30-600, 30-500, 30-400, 30-300, 30-200, 30-100, 40-1000, 40-900, 40-800, 40-700, 40-600, 40-500, 40-400, 40-300, 40-200, 40-100, 50-1000, 50-900, 50-800, 50-700, 50-600, 50-500, 50-400, 50-300, 50-200, 50-100, 60-1000, 60-900, 60-800, 60-700, 60-600, 60-500, 60-400, 60-300, 60-200, 60-100, 70-1000, 70-900, 70-800, 70-700, 70-600, 70-500, 70-400, 70-300, 70-200, 70-100, 80-1000, 80-900, 80-800, 80-700, 80-600, 80-500, 80-400, 80-300, 80-200, 80-100, 90-1000, 90-900, 90-800, 90-700, 90-600, 90-500, 90-400, 90-300, 90-200, 90-100, 100-1000, 100-900, 100-800, 100-700, 100-600, 100-500, 100-400, 100-300, or 100-photosensitive molecules. In some embodiments, the VLP may comprise 10, 25, 50, 75, 100, 125 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, 825, 950, 975, or 1000 photosensitive molecules. In some embodiments, the VLP may comprise more than 1000 (e.g., 1500, 2000, etc.) photosensitive molecules or less than 10 photosensitive molecules.

More than one photosensitive molecule may be conjugated to a single capsid protein. For example, a single capsid protein *(e.g.,* L1 or L2 capsid protein) may be conjugated to 1 to *5 (e.g.,* 1, 2, 3, 4 or 5) photosensitive molecules. Thus, more than one amino acids of a capsid protein may be conjugated to a photosensitive molecule. In some embodiments, a single capsid protein may be conjugated to 1 to 2, 1 to 3, or 2 to 3 photosensitive molecules. Thus, a photosensitive molecule may be conjugated to 1, 2, 3, 4 or 5 different amino acids *(e.g.,* lysine, arginine and/or histidine, or other amino acid) of a single capsid protein.

Examples of photosensitive molecules for use in accordance with the present disclosure include, without limitation, fluorescent dyes, infrared dyes, near infrared dyes, porphyrin molecules and chlorophyll molecules. The VLPs, in some embodiments, include a combination of photosensitive molecules, such as therapeutic photosensitizing dye molecules and non-toxic imaging (e.g., fluorescent) dye molecules.

Examples of photosensitizing dyes for use in accordance with the present disclosure include, without limitation, IRDye^{®} 700DX, HpD, Porfimer sodium( Photofrin@, Photogem^{®}, Photosan Hemporfin^{®}), m-THPC, Temoporfin (Foscan^{®}), Verteporfin (Visudyne^{®}), HPPH (Photochlor^{®}), Palladium-bacteria-pheophorbide (Tookad^{®},) 5-ALA, 5 aminolevulinic acid (Levulan^{®}), 5-ALA methylester (Metvix^{®}), 5-ALA benzylester (Benzvix^{®}), 5-ALA hexylester ( Hexvix@), lutetium (III)-texaphyrin or Motexafin-lutetium (Lutex^{®}, Lutrin^{®}, Angrin^{®}, Optrin^{®}), SnET2, Tin (IV) ethyl etiopurpurin (Purlytin^{®}, Photrex^{®}), NPe6, mono-L-aspartyl chlorine e6, talaporfin sodium (Talporfin^{®}, Laserphyrin^{®}), BOPP, boronated protoporphyrin (BOPP^{®}), Zinc phthalocyanine (CGP55847^{®}), silicon phthalocyanine (Pc4^{®}), mixture of sulfonated aluminium phthalocyanine derivatives (Photosens^{®}), ATMPn, Acetoxy-tetrakis (beta-methoxyethyl-)porphycene), TH9402 and dibromorhodamine methyl ester.

In some embodiments, the photosensitizing dye is the IRDye^{®} 700DX NHS ester having the chemical formula C₇₄H₉₆N₁₂Na₄O₂₇S₆Si₃ and dye structure:

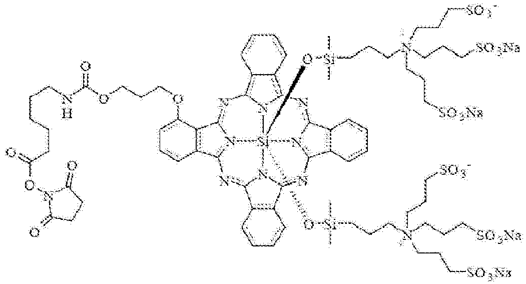

Examples of imaging dyes (e.g., fluorescent dyes) for use in accordance with the present disclosure include, without limitation, IRDye^{®} 800CW, acridine orange, acridine yellow, Alexa Fluor, 7-Aminoactinomycin D, 8-Anilinonaphthalene-1-sulfonic acid, ATTO dyes, auramine-rhodamine stain, benzanthrone, bimane, 9,10-Bis(phenylethynyl)anthracene, 5,12-Bis(phenylethynyl)naphthacene, bisbenzimide, blacklight paint, calcein, carboxyfluorescein, carboxyfluorescein diacetate succinimidyl ester, carboxyfluorescein succinimidyl ester, 1-chloro-9,10-bis(phenylethynyl)anthracene, 2-chloro-9,10-bis(phenylethynyl)anthracene, 2-chloro-9,10-diphenylanthracene, coumarin, DAPI, dark quencher, DiOC6, DyLight Fluor, Fluo-3, Fluo-4, FluoProbes, fluorescein, fluorescein isothiocyanate, fluorescence image-guided surgery, fluoro-jade stain, fura-2, fura-2-acetoxymethyl ester, GelGreen, GelRed, green fluorescent protein, heptamethine dyes, Indian yellow, Indo-1, Lucifer yellow, luciferin, MCherry, Merocyanine, Nile blue, Nile red, optical brightener, perylene, phloxine, phycobilin, phycoerythrin, phycoerythrobilin, propidium iodide, pyranine, rhodamine, rhodamine 123, Rhodamine 6G, RiboGreen, RoGFP, rubrene, (E)-stilbene, (Z)-stilbene, sulforhodamine 101, sulforhodamine B, SYBR Green I, synapto-pHluorin, tetraphenyl butadiene, tetrasodium tris(bathophenanthroline disulfonate)ruthenium(II), Texas Red, Titan yellow, TSQ, umbelliferone, yellow fluorescent protein and YOYO-1.

In some embodiments, the photosensitizing dye is the IRDye^{®} 800CW NHS ester having the chemical formula C₅₀H₅₄N₃Na₃O₁₇S₄ and dye structure:

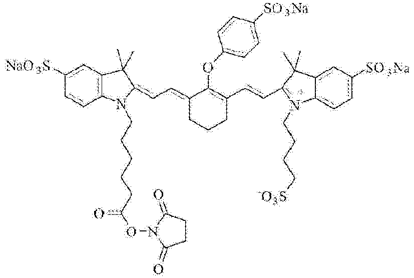

Photosensitive molecules of the disclosure can be activated at a suitable wavelength. In some embodiments, activation of the photosensitive molecules renders them cytotoxic or able to produce a cytotoxic molecule. Suitable wavelengths include, without limitation, ultraviolet wavelengths, visible wavelengths, infrared wavelengths and near infrared wavelengths. In some embodiments, the photosensitive molecules are activated and become cytotoxic at a wavelength of 600 nm to 800 nm, or 660 nm to 740 nm. In some embodiments, the photosensitive molecules are activated and become cytotoxic at a wavelength of about 600 nm, 610 nm, 620 nm, 630 nm, 640 nm, 650 nm, 660 nm, 670 nm, 680 nm, 690 nm, 700 nm, 710 nm, 720 nm, 730 nm, 740 nm, 750 nm, 760 nm, 770 nm, 780 nm, 790 nm or 800 nm. In some embodiments, the photosensitive molecules are activated at a wavelength of less than 600 nm or more than 800 nm. Suitable wavelengths for photosensitive molecule activation will depend on the particular molecule used.

### Methods of Production and Drug Conjugation

To produce VLPs drug conjugates of the present disclosure, mammalian cells, such as 293T cells *(e.g.,* HEK293F cells) may be grown *(e.g.,* in suspension culture) and transiently transfected with a nucleic acid (e.g., bicistronic plasmid DNA) encoding HPV L1 (or L1 and L2) capsid proteins. This induces the formation of protocapsids *(e.g.,* as described in Buck et. al. Current Protocols in Cell Biology 26.1.1-26.1.19, December 2007). Following cell mass recovery and disruption, the protocapsids may be subjected to host DNA clearance with benzonase treatment and a subsequent maturation process *in vitro* to form stable VLPs. Following purification, the VLPs may be chemically conjugated with photosensitive molecules (e.g., IR700 NHS ester) to produce the VLP drug conjugates.

In some embodiments, the VLP drug conjugate is prepared as follows: conjugation is performed with IRDye^{®} 700DX NHS Ester at a calculated molar excess of 300:1 (dye: VLP) for 2 hours following 1:1 dilution with 2X labelling buffer comprising 100 mM HEPES pH 7.5, 20 mM MgCl₂, and 10% (w/v) trehalose dihydrate. The total protein concentration, in some embodiments, is determined by the Bradford total protein assay (Pierce Bradford protein assay Cat. # 23200) according to the manufacturer's instructions using the micro-microplate procedure. The VLP drug conjugate, in some embodiments, is then buffer exchanged into a particular formulation.

Thus, in some aspects, provided herein are methods of producing photosensitive molecules, comprising (a) transiently transfecting cells with a nucleic acid that encodes one or more capsid proteins, thereby forming protocapsids, (b) collecting the protocapsids and subjecting the protocapsids to a maturation process *in vitro,* thereby forming stable VLPs, and (c) chemically conjugating the VLPs (capsids of the VLPs) to 10 to 1000 (e.g., 10-500, 50-1000, 50-500, 100-1000, 100-500, 100, 200, 300, 400, or 500) photosensitive molecules. In some embodiments, the VLPs are conjugated to photosensitive molecules through an amide bond *(e.g.,* by reacting an ester group of a photosensitive molecule with an amine group of an amino acid the capsid protein of a viral-like nanoparticle).

Conjugation may be by any method known in the art. Non-limiting examples of methods for conjugating photosensitive molecules to capsid proteins include reacting N-hydroxysuccinimide ester (NHS-ester)-labeled photosensitive molecules with amine groups on capsid proteins; reacting maleimide, iodoacetyl groups, or pyridyl disulfides-labeled photosensitive molecules with sulfhydryl groups on capsid proteins; and reacting primary amine-labeled photosensitive molecules with carboxyl groups on capsid proteins.

In some embodiments, conjugation includes reacting NHS-ester labeled-photosensitive molecules with amine groups on capsid proteins. Available amine groups are on the amino terminus of the capsid proteins or the ε-amino group on any lysine amino acid. In some embodiments, photosensitive molecules are conjugated to capsid proteins in a suitable buffer (*e.g.,* PBS, pH 7.2, 0.3M - 0.5 M NaCl). In some embodiments, photosensitive molecules and capsid proteins are mixed together at ratios of 1:200, 1:300, 1:400, 1:500, 1:600, 1:700, 1:800, 1:900, or 1:1000. The conjugation reactions, in some embodiments, are performed at room temperature.

VLPs and photosensitive molecules that are being conjugated may be exposed to a first light and a second light during conjugation. In some embodiments, the first light is as described herein (*e.g*., having a wavelength of 470-610 nm and an intensity of at least 500 lux). In some embodiments, the second light is as described herein (*e.g.*, white light having an intensity of at least 500 lux). Exposure to the first and/or second light during conjugation may be for ensuring that the photosensitive molecules are conjugated to the VLPs, collecting samples from the conjugation reaction for quality control, or for checking the conjugation reaction for homogeneity. Homogeneity refers to a well-mixed solution that is visibly free from particulates.

In some embodiments, VLPs and photosensitive molecules are exposed to the first light during conjugation for no more than 15 minutes. In some embodiments, VLPs and photosensitive molecules are exposed to the first light during conjugation for 2-8 minutes. In some embodiments, VLPs and photosensitive molecules are exposed to the first light during conjugation for 3-10 minutes.

In some embodiments, VLPs and photosensitive molecules are exposed to the second light during conjugation for no more than 15 minutes. In some embodiments, VLPs and photosensitive molecules are exposed to the second light during conjugation for 1-5 minutes. In some embodiments, VLPs and photosensitive molecules are exposed to the second light during conjugation for 3-10 minutes.

### Methods of Treatment

Any type of tumor can be targeted in accordance with the present disclosure. Examples of tumors include, without limitation, those located in the eye, lung, pleura, liver, pancreas, stomach, esophagus, colon, breast, ovary, prostate, brain, meninges, testis, kidneys, bladder, head, neck, cervix, larynx and/or skin. For example, the present application provides methods and compositions for targeting cervical cancer cells, ovarian cancer cells, melanoma cancer cells, lung cancer cells, head and/or neck cancer cells, and bladder cancer cells. Other tumors may also be targeted.

In some embodiments, the tumor is an ocular tumor or lesion. The ocular tumor or lesion may be located in the vitreous, choroidal space, suprachoroidal space, iris, ciliary body, sclera, fovea, retina, optic disk, or optic nerve. Thus, in some embodiments, a subject administered an ophthalmic composition of the present disclosure has an ocular tumor. The ocular tumor may be, for example, an uveal melanoma or a choroidal melanoma. The tumor, in some embodiments, is cancerous or malignant. In some embodiments, the tumor is metastatic. In some embodiments, lesion is a pre-cancerous lesion or an indeterminate lesion. In some embodiments, a subject administered an ophthalmic composition of the present disclosure has an indeterminate lesion.

In some embodiments, a subject administered an ophthalmic composition of the present disclosure has a choroidal metastasis, which originated from elsewhere in the body and spread to the eye. For example, the choroidal metastasis may have originated from a breast cancer or a lung cancer in men.

The ophthalmic compositions of the present disclosure are typically administered via intravitreal or suprachoroidal injection, although other routes of administration are contemplated herein.

In some embodiments, the ophthalmic composition (or any component thereof) is formulated as a solution. In some embodiments, the ophthalmic composition (or any component thereof) is lyophilized.

### Additional Embodiments

1. An ophthalmic composition comprising a near-isotonic solution of a virus-like particle (VLP) drug conjugates comprising photosensitive molecules conjugated to capsid proteins of a VLP, wherein the VLP drug conjugates are in suspension.
2. An ophthalmic composition comprising a virus-like particle (VLP) drug conjugates comprising photosensitive molecules conjugated to capsid proteins of a VLP, wherein the VLP drug conjugates do not aggregate to form visible particulate.
3. The ophthalmic composition of paragraph 1 or 2 having a pH value of less than 7.
4. The ophthalmic composition of any one of paragraphs 1-3 further comprising 2-(N-morpholino)ethanesulfonic acid (MES).
5. The ophthalmic composition of any one of paragraphs 1-4 further comprising at least one protective excipient and at least one detergent.
6. The ophthalmic composition of any one of paragraphs 1-4 further comprising at least one reagent selected from trehalose dihydrate, magnesium chloride (MgCl₂), sodium chloride (NaCl), and polysorbate 80 (PS80).
7. The ophthalmic composition of paragraph 6 further comprising at least two reagents selected from trehalose dihydrate, MgCl₂, NaCl, and PS80.
8. The ophthalmic composition of paragraph 7 further comprising at least three reagents selected from trehalose dihydrate, MgCl₂, NaCl, and PS80.
9. The ophthalmic composition of paragraph 8 further comprising trehalose dihydrate, MgCl₂, NaCl, and PS80.
10. The ophthalmic composition of any one of paragraphs 1-9, wherein the composition comprises 0.1% to 1.0% (w/v) MES or a pharmaceutically acceptable salt thereof (e.g.,. MES hemisodium salt).
11. The ophthalmic composition of paragraph 10, wherein the composition comprises 0.4% (w/v) MES.
12. The ophthalmic composition of any one of paragraphs 1-11, wherein the composition comprises 1 % to 10% (w/v) trehalose dihydrate.
13. The ophthalmic composition of paragraph 12, wherein the composition comprises 5% (w/v) trehalose dihydrate.
14. The ophthalmic composition of any one of paragraphs 1-13, wherein the composition comprises 0.1% to 1.0% (w/v) NaCl.
15. The ophthalmic composition of paragraph 14, wherein the composition comprises 0.4% (w/v) NaCl.
16. The ophthalmic composition of any one of paragraphs 1-15, wherein the composition comprises 0.1% to 1.0% (w/v) MgCl₂.
17. The ophthalmic composition of paragraph 16, wherein the composition comprises 0.2% (w/v) MgCl₂.
18. The ophthalmic composition of any one of paragraphs 1-17, wherein the composition comprises 0.01% to 0.1% (w/v) PS80.
19. The ophthalmic composition of paragraph 18, wherein the composition comprises 0.05% (w/v) PS80.
20. The ophthalmic composition of any one of paragraphs 1-19, wherein the composition comprises 0.01% to 0.5% (w/v) VLP drug conjugate.
21. The ophthalmic composition of paragraph 20, wherein the composition comprises 0.01% to 0.1% (w/v) VLP drug conjugate.
22. The ophthalmic composition of paragraph 21, wherein the composition comprises 0.04% VLP drug conjugate.
23. The ophthalmic composition of any one of paragraphs 3-22, wherein the composition has a pH value of 6.5.
24. An ophthalmic composition comprising 0.43% (w/v) 2-(N-morpholino)ethanesulfonic acid (MES), 5% (w/v) trehalose dihydrate, 0.37% (w/v) sodium chloride, 0.2% (w/v) magnesium chloride, 0.05% (w/v) polysorbate 80, and 0.04% (w/v) virus-like particle (VLP) drug conjugate, wherein the VLP drug conjugate comprises photosensitive molecules conjugated to capsid proteins of a VLP.
25. The ophthalmic composition of any one of paragraphs 1-24, wherein the photosensitive molecules comprise dye molecules.
26. The ophthalmic composition of paragraph 25, wherein the dye molecules comprise phthalocyanine dye molecules.
27. The ophthalmic composition of paragraph 26, wherein the phthalocyanine dye molecules comprise IRDye^{®} 700DX.
28. The ophthalmic composition of any one of paragraphs 1-27, wherein the VLPs comprise 10-1000 photosensitive molecules, 10-500 photosensitive molecules, 50-1000 photosensitive molecules, 50-500 photosensitive molecules, 100-1000 photosensitive molecules, or 100-500 photosensitive molecules.
29. The ophthalmic composition of any one of paragraphs 1-28, wherein the VLPs comprise 50-500 photosensitive molecules.
30. The ophthalmic composition of any one of paragraphs 1-29, wherein the VLP comprises papillomavirus capsid proteins.
31. The ophthalmic composition of paragraph 30, wherein the papillomavirus capsid proteins are human papillomavirus capsid proteins.
32. The ophthalmic composition of paragraph 31, wherein the papillomavirus capsid proteins comprise L1 capsid proteins, L2 capsid proteins, or a combination of L1 and L2 capsid proteins.
33. The ophthalmic composition of paragraph 32, wherein the L1 capsid proteins are modified to reduce immunogenicity of the VLP.
34. A method comprising administering to an eye of a subject the ophthalmic solution of any one of paragraphs 1-33, wherein the subject has ocular melanoma, and wherein the ophthalmic solution is administered in an amount effective to treat the ocular melanoma.
35. The method of paragraph 34, wherein the ocular melanoma is an uveal melanoma or a choroidal melanoma.
36. A method comprising administering to an eye of a subject the ophthalmic solution of any one of paragraphs 1-33, wherein the subject has an indeterminate lesion, and wherein the ophthalmic solution is administered in an amount effective to treat the indeterminate lesion.
37. The method of any one of paragraphs 34-36, wherein the ophthalmic composition is injected intravitreally.
37. A method comprising administering to an eye of a subject the ophthalmic solution of any one of claims 1-33, wherein the subject has a choroidal metastasis, and wherein the ophthalmic solution is administered in an amount effective to treat the indeterminate lesion.
38. The method of any one of paragraphs 34-37, wherein the ophthalmic composition is injected intravitreally.
39. The method of any one of paragraphs 34-37, wherein the ophthalmic composition is injected into the suprachoroidal space of the eye.
40. The method of paragraph 39, wherein the ophthalmic composition remains in the suprachoroidal space of the eye for at least 1 week.
41. The method of paragraph 39 or 40, wherein white blood cell infiltrate is not observed in the ciliary body and/or sclera following at least 35 days following injection of the ophthalmic composition.
42. The method of any one of paragraphs 34-41, wherein optical coherence tomography is normal in the eye of the subject following injection of the ophthalmic composition.
43. The method of any one of claims 34-42, wherein intraocular pressure is normal in the eye of the subject following injection of the ophthalmic composition.
44. The ophthalmic composition of any one the foregoing paragraphs, wherein the ophthalmic composition is DNase and/or RNase free.
45. The ophthalmic composition of any one the foregoing paragraphs, wherein the ophthalmic composition is sterile.

All references, patents and patent applications disclosed herein are incorporated by reference with respect to the subject matter for which each is cited, which in some cases may encompass the entirety of the document.

The indefinite articles "a" and "an," as used herein in the specification and in the claims, unless clearly indicated to the contrary, should be understood to mean "at least one."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

The terms "about" and "substantially" preceding a numerical value mean ±10% of the recited numerical value.
Where a range of values is provided, each value between the upper and lower ends of the range are specifically contemplated and described herein.

### EXAMPLES

In the initial evaluation of VLP drug conjugate formulations, a historical phosphate-based high salt formulation and three alternate formulations were tested to evaluate in-process recovery upon formulation of the bulk VLP drug conjugate as well as the parameters of the formulated materials in response to repeated freeze-thaw cycles. The data collected included protein recovery by the Bradford total protein assay, A280, SDS-PAGE banding pattern, VLP morphology by transmission electron microscopy, VLP size distribution by dynamic light scattering, VLP drug conjugate potency, ocular distribution of the VLP conjugate following suprachoroidal injection, and VLP drug conjugate safety *in vivo.*

The data below shows unexpectedly that VLP drug conjugate recovery was increased upon a change in product formulation to a MES-based formulation containing 5% trehalose dehydrate, without evidence of visible aggregation during or following excess dye removal using tangential flow filtration (TFF).

### Example 1: Process Step Recovery

A bulk VLP drug conjugate was prepared by buffer exchange and concentration using tangential flow filtration (TFF) into each of four formulations (A, B, C, D). The control comprised the historical phosphate/high salt based formulation. Three additional trehalose-containing formulations were evaluated with the intent that the combination of buffer pH and trehalose excipient would protect the bioconjugate. Observations collected during the TFF procedure included the formation of visible aggregation in the PBS/High salt control formulation and the phosphate-based and trehalose/PS80 containing formulation A. No evidence of aggregation was seen for the HEPES or MES-based formulations containing trehalose and PS80 (Formulations B and C, respectively). Step recoveries were determined using the Bradford total protein assay. The two phosphate-based formulations (where evidence of aggregation were seen) resulted in ~48-58% step recovery, significantly lower than the recovery in HEPES (69.7%) and the MES-based formulation (79.3%) **(Table 1).** The concentrations of the final formulated samples were between 0.25 and 0.66 mg/mL. To avoid further processing manipulation and sample losses, a decision was made to evaluate the properties of these samples without further processing.

**Table 1: Step-Recovery by Bradford Total Protein Assay**

| **Input Bulk Bio conjugate (mg)** | **Final Formulation** | **Output Bulk VLP Drug Conjugate (mg)** | **% Recovery** | **Observation** |
|---|---|---|---|---|
| 2.08 | Control | 1.2 | 57.6 | **Precipitation** observed during buffer exchange |
| 2.08 | A | 1.0 | 48.0 | **Precipitation** upon buffer exchange and concentration |
| 2.08 | B | 1.5 | 69.7 | No precipitation |
| 2.08 | C | 1.6 | 79.3 | No precipitation |

### Example 2: Recovery Following Freeze-Thaw

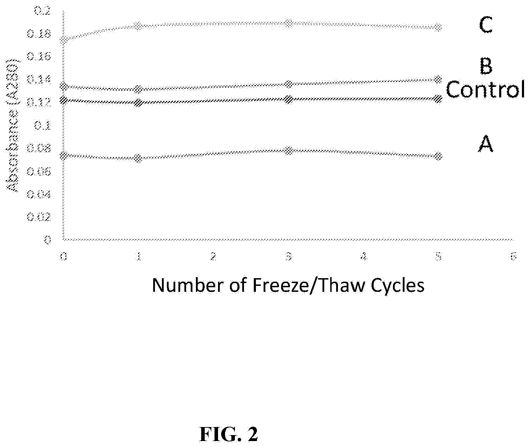

Samples were filled into 2.0mL CZ resin vials. A number of vials from each formulation were stored at 2-8°C, while additional vials were frozen. Samples were subjected to one, three or five freeze-thaw cycles and the Bradford assay or UV-VIS was used to determine total protein recoveries. This data is presented below in **Table 2** and graphically in **FIGS. 1-2****.**

**Table 2: Recovery by Bradford Total Protein Assay**

| | **Formulation/Protein concentration (mg/mL)** | | | |
|---|---|---|---|---|
| **Freeze Thaw Cycles** | **Control (mg/mL)** | **A (mg/mL)** | **B (mg/mL)** | **C (mg/mL)** |
| 0 FT | 0.34 | 0.25 | 0.45 | 0.66 |
| 1 FT | 0.36 | 0.21 | 0.42 | 0.52 |
| 3 FT | 0.37 | 0.27 | 0.47 | 0.82 |
| 5 FT | 0.37 | 0.25 | 0.46 | 0.61 |

Within the error of the analytical assay (expected ~ 20%) used there was no apparent loss of protein irrespective of final formulation condition or number of freeze-thaw cycles. A consistent recovery was also obtained from each sample when analyzed by UV-VIS at 280nm to follow total protein recovery.

### Example 3: Evaluation by SDS-PAGE

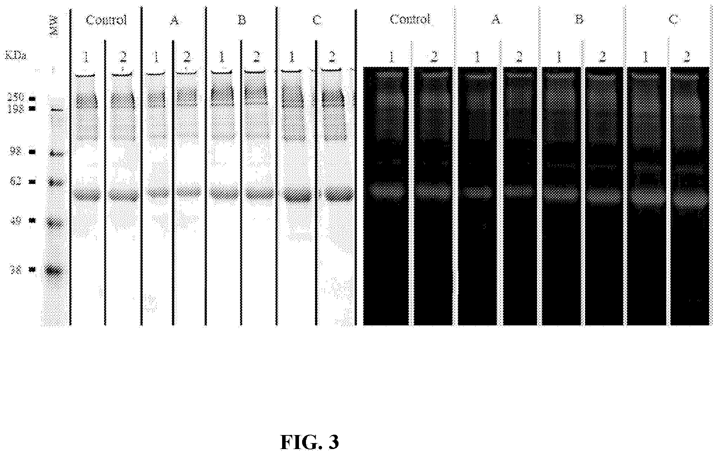

Samples from each formulation were analyzed by SDS-PAGE and the gel imaged by standard Coomassie staining and also by fluorescence, using an Odyssey scanner to detect the fluorescence of the DX700 dye. Freshly prepared samples and those subjected to one, three or five cycles of freeze-thaw were analyzed. Samples were loaded by volume. A representative gel image showing the Coomassie stained gel and a fluorescence scan of the same gel for samples having undergone five freeze-thaw cycles is shown in **FIG. 3****.** Visual evaluation of the SDS-PAGE gel reveals a complex banding pattern characteristic of the VLP drug conjugate. A predominant band that is the L1 protein can be seen migrating at approximately 55 kDa. A faint band can also be seen above the 62 kDa marker and represents the L2 protein. Above the 98 kDa marker, a ladder of species is seen indicating some form of oligomeric material is present. The nature of this material is the subject of other technical reports and LC-MS analysis of in-gel digestion samples performed by SGS reveals that this material is protein comprising L1 and L2 sequences. Visually, the banding patterns are consistent across all samples and the band intensities and fluorescent signals can be seen to parallel the Bradford and Absorbance datasets.

### Example 4: Virus-like particle morphology and size distribution by Transmission Electron Microscopy

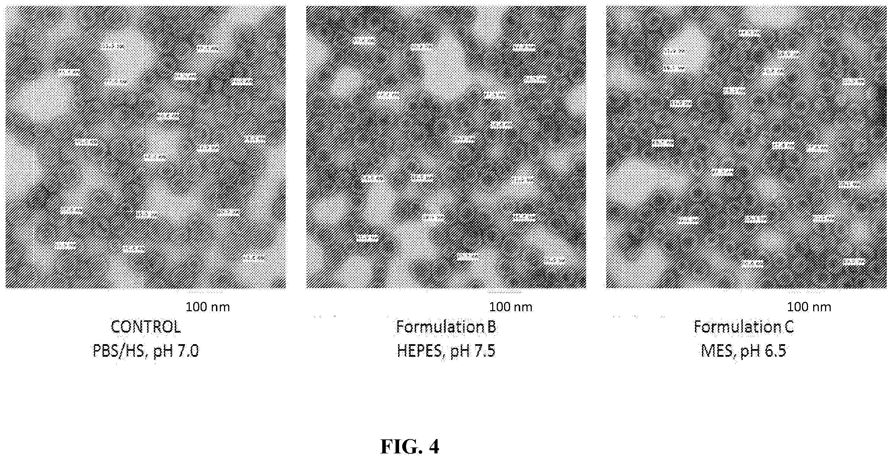

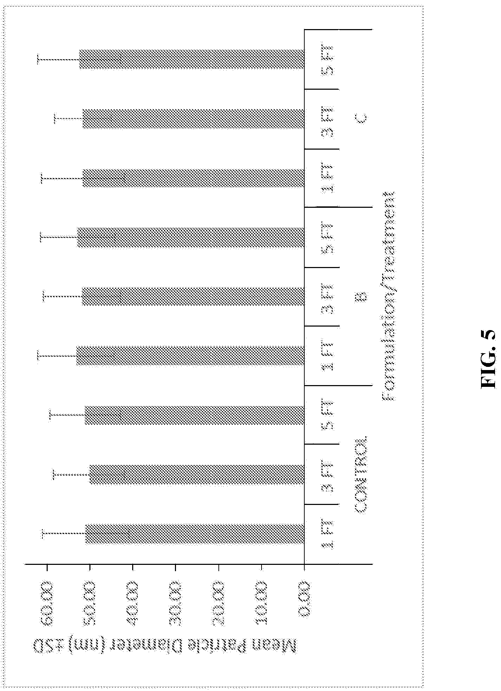

The size of the particles were estimated by measuring the diameter of ~60-90 particles and assembled capsomer material using AMT software. Most of the VLPs appear to be fully assembled, are observed to have a range of sizes, and are predominantly spherical or ellipsoidal in shape **FIG. 4****.** Similar ranges in apparent morphology have been observed in EM studies of HPV16 L1 and HPV11 L1 based VLPs as well as in unfractionated preparations of rabbit papillomavirus virus. TEM data is represented in **Table 3** and graphically in **FIG. 5** for the samples subjected to one, three and five cycles of freeze-thaw. During the freeze-thaw stability study, no significant difference was observed in particle size of VLP drug conjugate between the three buffer conditions evaluated - Control, Formulation B and Formulation C. Formulation A was not evaluated using TEM. No significant change in VLP drug conjugate size-range distribution or gross morphological properties was observed through multiple freeze-thaw cycles during the study.

**Table 3: Particle Size Data via TEM**

| **Formulation** | **CONTROL** | | | **B** | | | **C** | | |
|---|---|---|---|---|---|---|---|---|---|
| **Treatment** | **1 FT** | **3 FT** | **5 FT** | **1 FT** | **3 FT** | **5 FT** | **1 FT** | **3 FT** | **5 FT** |
| **Mean (nm)** | 50.86 | 50.11 | 51.02 | 56.57 | 52.21 | 53.26 | 54.67 | 51.55 | 54.22 |
| **SD** | 10.02 | 8.16 | 8.27 | 6.43 | 8.49 | 8.13 | 7.23 | 6.58 | 8.15 |
| **Range (min/max)** | 32.7/76.7 | 31.6/70.3 | 31.6/68.8 | 32.1/74.2 | 32.3/67.4 | 30.5/66 | 30.5/71.2 | 32/67.6 | 31.4/71 |

### Example 5: Particle Size Analysis Using Dynamic Light Scattering

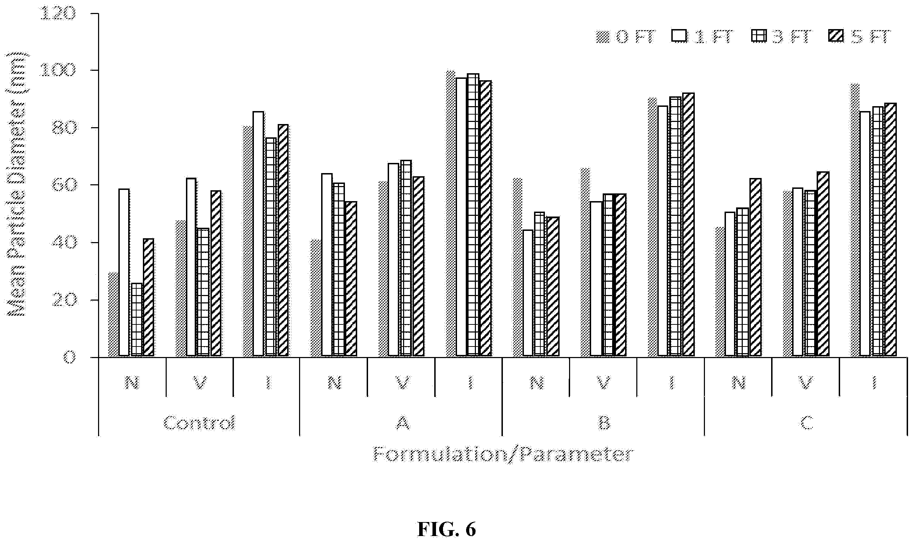

To complement the morphology and size distribution analysis by TEM, the average sizes of VLPs in each formulation were estimated by DLS assay before being frozen and following one, three or five freeze-thaw cycles. Laser-based DLS can monitor changes in the motion and structure of nanoparticles in solution (i.e. degradation or VLP-oligomerization) and provides information related to the average size and frequency distribution of particles. DLS data is summarized in **Table 4 and** presented graphically in **FIG. 6****.** In both, the values for mean diameter by number (N), volume (V) and intensity (I) are shown. Each gives a snapshot of the average particle size distribution and reports an average value for entire distribution. Irrespective of the formulation and number of freeze-thaw cycles, there was no apparent change seen in the average particle size distribution. Together, the DLS and TEM measurements of VLPs were consistent and show the VLPs to be of comparable size and morphology independent of formulation and number of freeze-thaw cycles.

**Table 4: Average Particle Size via DLS**

| | **Formulation/Average particle size (nm)** | | | | | | | | | | | |
|---|---|---|---|---|---|---|---|---|---|---|---|---|
| | **Control** | | | **A** | | | **B** | | | **C** | | |
| # F/T | N | V | I | N | V | I | N | V | I | N | V | I |
| 0 | 29.5 | 47.6 | 80.7 | 40.8 | 61.3 | 99.7 | 62.5 | 65.8 | 90.3 | 45.5 | 57.8 | 95.3 |
| 1 | 57.7 | 61.7 | 85.1 | 62.8 | 66.4 | 96.8 | 43.9 | 53.5 | 86 | 49.5 | 57.4 | 84.4 |
| 3 | 25.9 | 43.8 | 76.1 | 59.9 | 68.0 | 97.8 | 49.2 | 56.2 | 89.5 | 50.6 | 57.1 | 87.0 |
| 5 | 40.3 | 57.3 | 80.4 | 53.2 | 62.5 | 96.2 | 48.2 | 56.5 | 91.0 | 60.6 | 64.0 | 87.8 |

### Example 6: Potency Evaluations

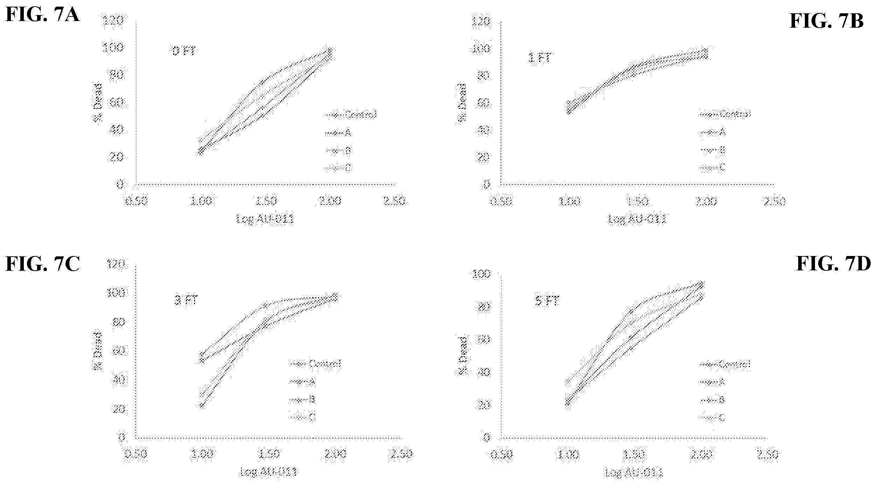

The functional properties of the VLP drug conjugate were evaluated using an *in vitro* killing assay. Data generated in each of the four tested formulations of freshly prepared material and material stored in the final container closure and subject to one, three or five freeze-thaw cycles is shown in **FIGS. 7A-7D****.** The limited capacity of staffing and the assay itself limited data points to one either side of the expected EC50. Irrespective of the formulation and number of freeze-thaw cycles, the VLP drug conjugate remained potent.

### Example 7: Ocular Distribution of VLP Drug Conjugates After Suprachoroidal Injection in New Zealand White Rabbit

The *in vivo* ocular distribution of VLP conjugates was evaluated by injecting AlexaFluor488*VLP into the suprachoroidal space (SCS) in New Zealand White (NZW) rabbits (Study PK-RPE-003). AlexaFluor488*VLP was used in place of the VLP drug conjugate because it has similar physiochemical characteristics and is formulated in the same MES buffer as the VLP drug conjugate, but is more suitable for *in vivo* imaging. Ocular distribution was evaluated with Optical Coherence Tomography (OCT) and Fundus Autofluorescence (FAF) over time. Data showed that the distribution after a 100 µl injection into the SCS was about 75% of the posterior globe at ≤0.5 hours post-dose and remained relatively constant over the duration of the study. Fluorescence was strong through the 168-hour post-dose interval and started to fade at the 240-hour post-dose interval. At the 504-hour post-dose interval, significant fluorescence over baseline could not be detected. This data suggests that the VLP drug conjugate formulated in MES buffer distributes well into the SCS space and that the duration is at least 168 hours (1 week).

### Example 8: In vivo Safety Evaluation of VLP Drug Conjugates (Nonclinical)

To evaluate the nonclinical safety of VLP drug conjugates, the VLP drug conjugate was administered in a dog study. The VLP drug conjugate was administered at a single dose-level via suprachoroidal space (SCS) 100 µ injection at the dose of 20 µg/eye followed by laser photoactivation 6-8 hours post injection to dogs. Dogs were treated once per week for 3 weeks (a total of 3 injections followed by laser treatment of 50 J/cm² 6-8 hours post-injection for each week). After the 3^{rd} weekly treatment , animals were observed for 7 days (terminal sacrifice) or 35 days (recovery phase) to assess the reversibility, persistence, or delayed occurrence of effects. There were only minor VLP drug conjugate related microscopic ocular findings. Histopathology showed minimal/slight white blood cell infiltrate into choroidal space and 50% of animals had minimal white blood cell infiltrate into ciliary body and sclera. The minimal/slight white blood cell infiltrates resolved between the first observation time point (terminal sacrifice animals at 7 days) and the second observation time point (recovery phase animals at 35 days) where it was not observed. The optical coherence tomography (OCT) was normal in all animals (i.e., no retinal pigment epithelium (RPE)/retinal changes or retinal thinning was detected) and all study eyes retained normal retinal structure. Intraocular pressure (IOP) of subject animals was evaluated during the course of the study and was normal for all animals. No systemic clinical findings, no change in body weight, and no findings on clinical pathology were reported. Together, the data suggests that VLP drug conjugate formulated in MES buffer delivered as multiple SCS injections is safe *in vivo.*

### Materials and Methods

### Samples

The VLP drug conjugate was formulated using four different formulations as outlined in **Table 5.**

**Table 5: Sample Formulations**

| **Description** | **Test Formulation** |
|---|---|
| Control | 20 mM Potassium Phosphate, 500 mM NaCl, pH 7.0 |
| A | 20 mM Potassium Phosphate, 5% trehalose dihydrate, 10 mM MgCl₂, 63 mM NaCl, 0.05% PS80, pH 7.0 |
| B | 50 mM HEPES, 5% trehalose dihydrate, 10 mM MgCl₂, 47 mM NaCl, 0.05% PS80, pH 7.5 |
| C | 20 mM MES, 5% trehalose dihydrate, 10 mM MgCl₂, 63 mM NaCl, 0.05% PS80, pH 6.5 |

### Equipment

The equipment necessary for conducting the formulation steps described herein is provided in **Table 6.**

**Table 6: Equipment**

| **Description** | **Manufacturer** |
|---|---|
| 1010 AMT camera | Jeol USA |
| Odyssey CLx | LiCor |
| NanoDrop 2000c | Thermo Scientific |
| FluorChem Gel Imager | Cell Biosciences |
| Synergy HT Microplate Reader | Biotek |
| Zeta PALS (DLS) | Brookhaven Instruments Corp. |

### Reagents and Solvents

Alcian Blue, 1.5% Uranyl Acetate, RO-DI water, Carbon film 300 Mesh Copper Grids, Fine-tip tweezers, Pipettes, Petri Dish, Parafilm, Filter Paper.

### VLP Drug Conjugate Manufacture and Formulation

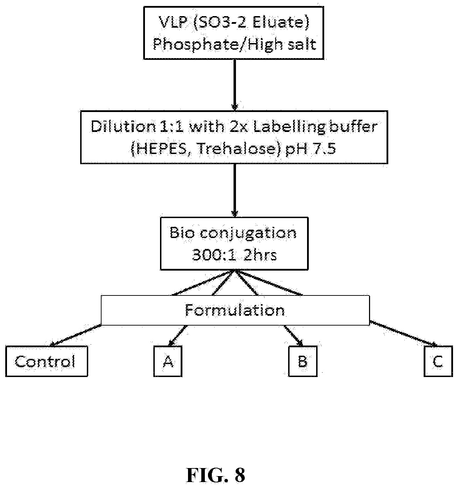

The VLP drug conjugate was prepared from bulk VLP preparation. Bioconjugation was performed with DX700-NHS Ester at a calculated molar excess of 300:1 (dye: VLP) for 2 hours following 1:1 dilution with 2X labelling buffer comprising 100 mM HEPES pH 7.5, 20 mM MgCl2, 10% trehalose dihydrate. The total protein concentration was determined by the Bradford total protein assay (Pierce Bradford protein assay Cat. # 23200) according to the manufacturer's instructions using the micro-microplate procedure). The stock VLP drug conjugate was then buffer exchanged into a control formulation (Phosphate/high salt) and three candidate formulations each containing trehalose and PS80 at three different pH's (A, B and C). The overall experimental flow is shown in FIG. 8.

### Product Strength

VLP drug conjugate product strength was determined by the Bradford total protein assay (Pierce Bradford protein assay Cat. # 23200 according to the manufacturer's instructions for the Micro-microplate format. A280nm was performed on undiluted samples using a nanodrop UV-VIS spectrophotometer blanked with sample matched buffers.

### SDS-PAGE Analysis

SDS-PAGE analysis of the VLP drug conjugate product was performed using a TGX criterion, Any kD gel (Bio-Rad #5671125). Standard gel images were captured using an imaging camera and fluorescence images were captured by scanning the gel on the Odyssey CLx. Fluorescence analysis was performed using Imagestudio software on Odyssey CLx.

### Dynamic Light Scattering

50 µL of the test sample was added to 150 µL of filtered PBS (Phosphate buffer saline, Boston Bioproducts Cat# BM220-S) and mixed gently by pipetting up and down 5 times. The diluted sample was then pipetted into a plastic cuvette (Fisher Cat# 14-955-125) and placed the cuvette in the DLS sample holder. The sample was analyzed using 90 Plus particle sizing software (Brookhaven Instruments). Briefly, the diluted sample was run three times, 2 minutes per run, at 25 °C with a 90° detector angle. Four separate distributions were recorded: Number, Volume, Intensity, and Surface Area. The polydispersity index of each sample run was also recorded to determine uniformity of the sample run.

### Transmission Electron Microscopy

Aliquots of VLP drug conjugate samples prepared in the different buffers were prepared and provided to Northeastern University electron microscopy center either unfrozen (2-8°C) or stored frozen at -80°C and subsequently thawed 1, 3 or 5 times before analysis. Samples were analyzed by TEM as follows: A carbon coated EM grid (Carbon film 300 mesh copper grid) was placed on top of a drop of alcian blue dye (placed on a parafilm section) for 30 seconds with the carbon side up. The excess dye was removed by gentle blotting using a filter paper. The EM grid was washed with deionized sterile water by gently grazing the grid surface on top of a water droplet (placed on a parafilm section) for 1-2 sec. The washing was repeated two more times using a fresh water drop for each wash. Upon washing, the grid was placed on top of a 10 µl drop of sample suspension for about 1 min. The loaded grid was gently blotted to remove excess sample followed by three washes with deionized water using the same technique as explained in step 2. The loaded grid was then stained by gentle grazing on the surface of a drop of 1.5% uranyl acetate stain for 1-2 sec and blotted with filter paper. The staining step was repeated three more times on the same drop of uranyl acetate. For TEM Imaging, a Jeol 1010 AMT camera was used (located at Electron Microscopy Center at Northeastern University, Boston). The prepared grid was placed into the TEM after few minutes of drying. The images were captured at a magnification of x15000, x25000, x30000, x40000, x50000, and x200000. The diameters of particles and assembled capsomers within a region of interest were measured at x40000 magnification by using the AMT software.

### Potency

The *in vitro* efficacy of the VLP drug conjugate was determined by performing a cell-killing assay (Aura-SOP-008) on the tumor cell line OCM-1. Due to sample and capacity limitations, only three product levels were evaluated near the expected EC50 of the product. Tumor cells were incubated with the VLP drug conjugate at various concentrations on ice for 1.5 hours. After this incubation, cells were washed to remove unbound VLP drug conjugate and subsequently re-suspended in sample diluent. Half of the VLP drug conjugate-bound cells for each VLP drug conjugate dilution were distributed to 3 wells of a 96-well plate (1/2 well, black, clear bottom) and irradiated with 25 J/cm2 of 690 nm wavelength light. Cells not irradiated served as controls.
**Embodiments of the invention may include the features of the following enumberated paragraphs** ("paras").
1. An ophthalmic composition comprising a near-isotonic solution of a virus-like particle (VLP) drug conjugates comprising photosensitive molecules conjugated to capsid proteins of a VLP, wherein the VLP drug conjugates are in suspension.
2. An ophthalmic composition comprising a virus-like particle (VLP) drug conjugates comprising photosensitive molecules conjugated to capsid proteins of a VLP, wherein the VLP drug conjugates do not aggregate to form visible particulate.
3. The ophthalmic composition of para 1 or 2 having a pH value of less than 7.
4. The ophthalmic composition of any one of paras 1-3 further comprising 2-(N-morpholino)ethanesulfonic acid (MES).
5. The ophthalmic composition of any one of paras 1-4 further comprising at least one protective excipient and at least one detergent.
6. The ophthalmic composition of any one of paras 1-4 further comprising at least one reagent selected from trehalose dihydrate, magnesium chloride (MgCl₂), sodium chloride (NaCl), and polysorbate 80 (PS80).
7. The ophthalmic composition of para 6 further comprising at least two reagents selected from trehalose dihydrate, MgCl₂, NaCl, and PS80.
8. The ophthalmic composition of para 7 further comprising at least three reagents selected from trehalose dihydrate, MgCl₂, NaCl, and PS80.
9. The ophthalmic composition of para 8 further comprising trehalose dihydrate, MgCl₂, NaCl, and PS80.
10. The ophthalmic composition of any one of paras 1-9, wherein the composition comprises 0.1% to 1.0% (w/v) MES.
11. The ophthalmic composition of para 10, wherein the composition comprises 0.4% (w/v) MES.
12. The ophthalmic composition of any one of paras 1-11, wherein the composition comprises 1 % to 10% (w/v) trehalose dihydrate.
13. The ophthalmic composition of para 12, wherein the composition comprises 5% (w/v) trehalose dihydrate.
14. The ophthalmic composition of any one of paras 1-13, wherein the composition comprises 0.1% to 1.0% (w/v) NaCl.
15. The ophthalmic composition of para 14, wherein the composition comprises 0.4% (w/v) NaCl.
16. The ophthalmic composition of any one of paras 1-15, wherein the composition comprises 0.1% to 1.0% (w/v) MgCl₂.
17. The ophthalmic composition of para 16, wherein the composition comprises 0.2% (w/v) MgCl₂.
18. The ophthalmic composition of any one of paras 1-17, wherein the composition comprises 0.01% to 0.1% (w/v) PS80.
19. The ophthalmic composition of para 18, wherein the composition comprises 0.05% (w/v) PS80.
20. The ophthalmic composition of any one of paras 1-19, wherein the composition comprises 0.01% to 0.5% (w/v) VLP drug conjugate.
21. The ophthalmic composition of para 20, wherein the composition comprises 0.01% to 0.1% (w/v) VLP drug conjugate.
22. The ophthalmic composition of para 21, wherein the composition comprises 0.04% (w/v) VLP drug conjugate.
23. The ophthalmic composition of any one of paras 3-22, wherein the composition has a pH value of 6.5.
24. An ophthalmic composition comprising 0.43% (w/v) 2-(N-morpholino)ethanesulfonic acid (MES), 5% (w/v) trehalose dihydrate, 0.37% (w/v) sodium chloride, 0.2% (w/v) magnesium chloride, 0.05% (w/v) polysorbate 80, and 0.04% (w/v) virus-like particle (VLP) drug conjugate, wherein the VLP drug conjugate comprises photosensitive molecules conjugated to capsid proteins of a VLP.
25. The ophthalmic composition of any one of paras 1-24, wherein the photosensitive molecules comprise dye molecules.
26. The ophthalmic composition of para 25, wherein the dye molecules comprise phthalocyanine dye molecules.
27. The ophthalmic composition of para 26, wherein the phthalocyanine dye molecules comprise IRDye^{®} 700DX.
28. The ophthalmic composition of any one of paras 1-27, wherein the VLPs comprise 10-1000 photosensitive molecules, 10-500 photosensitive molecules, 50-1000 photosensitive molecules, 50-500 photosensitive molecules, 100-1000 photosensitive molecules, or 100-500 photosensitive molecules.
29. The ophthalmic composition of any one of paras 1-28, wherein the VLPs comprise 50-500 photosensitive molecules.
30. The ophthalmic composition of any one of paras 1-29, wherein the VLP comprises papillomavirus capsid proteins.
31. The ophthalmic composition of para 30, wherein the papillomavirus capsid proteins are human papillomavirus capsid proteins.
32. The ophthalmic composition of para 31, wherein the papillomavirus capsid proteins comprise L1 capsid proteins, L2 capsid proteins, or a combination of L1 and L2 capsid proteins.
33. The ophthalmic composition of para 32, wherein the L1 capsid proteins are modified to reduce immunogenicity of the VLP.
34. A method comprising administering to an eye of a subject the ophthalmic solution of any one of paras 1-33, wherein the subject has ocular melanoma, and wherein the ophthalmic solution is administered in an amount effective to treat the ocular melanoma.
35. The method of para 34, wherein the ocular melanoma is an uveal melanoma or a choroidal melanoma.
36. A method comprising administering to an eye of a subject the ophthalmic solution of any one of paras 1-33, wherein the subject has an indeterminate lesion, and wherein the ophthalmic solution is administered in an amount effective to treat the indeterminate lesion.
37. A method comprising administering to an eye of a subject the ophthalmic solution of any one of paras 1-33, wherein the subject has a choroidal metastasis, and wherein the ophthalmic solution is administered in an amount effective to treat the indeterminate lesion.
38. The method of any one of paras 34-37, wherein the ophthalmic composition is injected intravitreally.
39. The method of any one of paras 34-37, wherein the ophthalmic composition is injected into the suprachoroidal space of the eye.
40. The method of para 39, wherein the ophthalmic composition remains in the suprachoroidal space of the eye for at least 1 week.
41. The method of **para** 39 or 40, wherein white blood cell infiltrate is not observed in the ciliary body and/or sclera following at least 35 days following injection of the ophthalmic composition.
42. The method of any one of **paras** 34-41, wherein optical coherence tomography is normal in the eye of the subject following injection of the ophthalmic composition.
43. The method of any one of paras 34-42, wherein intraocular pressure is normal in the eye of the subject following injection of the ophthalmic composition.

## Claims

1. An ophthalmic composition for use in a treatment for an ocular cancer, wherein:
(a) the ophthalmic composition comprises a near-isotonic solution of a virus-like particle (VLP) drug conjugate comprising photosensitive molecules conjugated to capsid proteins of the VLP, wherein the VLP drug conjugate is in suspension, the near-isotonic solution has an isotonicity of 255-345 mOsm/L, and the ophthalmic composition comprises 0.2% to 0.4% w/v NaCl; and
(b) the use comprises (i) administering the ophthalmic composition to an eye of a subject who has the ocular cancer and (ii) activating the photosensitive molecules.

2. The ophthalmic composition for use according to claim 1, having a pH value of less than 7, optionally a pH value of 6.5.

3. The ophthalmic composition for use according to claim 1 or 2, further comprising 2-(N-morpholino)ethanesulfonic acid (MES).

4. The ophthalmic composition for use according to claim 3, comprising 0.1% to 1.0% (w/v) MES.

5. The ophthalmic composition for use according to claim 3, comprising 0.4% (w/v) MES.

6. The ophthalmic composition for use according to any one of claims 1-5, further comprising trehalose dihydrate, magnesium chloride (MgCl₂), and polysorbate 80 (PS80).

7. The ophthalmic composition for use according to any one of claims 1-6, comprising 1% to 10% (w/v) trehalose dihydrate, optionally 5% (w/v) trehalose dihydrate.

8. The ophthalmic composition for use according to any one of claims 1-7, comprising 0.1% to 1.0% (w/v) MgCl₂, optionally 0.2% (w/v) MgCl₂.

9. The ophthalmic composition for use according to any one of claims 1-8, comprising 0.01% to 0.1% (w/v) PS80, optionally 0.05% (w/v) PS80.

10. The ophthalmic composition for use according to any one of claims 1-9, comprising 0.4% (w/v) NaCl.

11. The ophthalmic composition for use according to any one of claims 1-10, comprising 0.01% to 0.5% (w/v) VLP drug conjugate, optionally 0.01% to 0.1% (w/v) VLP drug conjugate, and further optionally 0.04% (w/v) VLP drug conjugate.

12. The ophthalmic composition for use according to any one of claims 1-11, wherein the photosensitive molecule comprise phthalocyanine dye molecules.

13. The ophthalmic composition for use according to claim 12, wherein the phthalocyanine dye molecules comprise IRDye^{®} 700DX.

14. The ophthalmic composition for use according to any one of claims 1-13, wherein the VLP drug conjugate comprises 50-500 photosensitive molecules.

15. The ophthalmic composition for use according to any one of claims 1-14, wherein the VLP drug conjugate comprises human papillomavirus capsid proteins, optionally selected from L1 and L2 capsid proteins.
